# EUROPÄISCHE PATENTSCHRIFT

(11) **EP 0 491 282 B1**
(45) Veröffentlichungstag und Bekanntmachung des Hinweises auf die Patenterteilung: **10.02.1999**
(21) Anmeldenummer: 91121329.6
(22) Anmeldetag: 12.12.1991
(51) Int. Cl.: C07D 475/04, C07D 475/08, C07K 2/00, G01N 33/82, A61K 31/525

(54) **Pteridin-Derivate, Verfahren zu ihrer Herstellung sowie deren Verwendung zur Herstellung von Immunogenen**
Pteridin derivatives, process for their preparation and their use in the preparation of immunogens
Dérivés de ptéridine, procédé pour leur préparation ainsi que leur utilisation dans la préparation d'immunogènes

(30) Priorität: 19.12.1990 DE 4040651
(43) Veröffentlichungstag der Anmeldung: 24.06.1992
(73) Patentinhaber: BOEHRINGER MANNHEIM GMBH, 68298 Mannheim (DE)
(72) Erfinder: Huber, Erasmus, Dr. rer. nat., W-8911 Unterfinning (DE); Klein, Christian, Dr., W-8120 Weilheim (DE); Baumgarten, Horst, Dr. rer. nat., W-8122 Penzberg (DE); Pappert, Gunter, Dr., W-8132 Tutzing (DE); Zink, Bruno, W-8114 Uffing (DE)

(56) Entgegenhaltungen:
- DE-A- 3 520 896
- GB-A- 653 068
- US-A- 2 515 483
- US-A- 2 540 274
- JOURNAL OF MEDICINAL CHEMISTRY Bd. 25, Nr. 2, Januar 1982, WASHINGTON Seiten 161 - 166; CARROLL TEMPLE, JR. ET. AL.: 'Synthesis of Pseudo Cofactor Analogues as Potential Inhibitors of the Folate Enzymes.'

## Beschreibung

Die vorliegende Erfindung bezieht sich auf neue Pteridin-Derivate, Verfahren zu deren Herstellung sowie deren Verwendung zur Herstellung von Immunogenen.

Gegenstand der vorliegenden Erfindung sind Pteridin-Derivate der Formel I 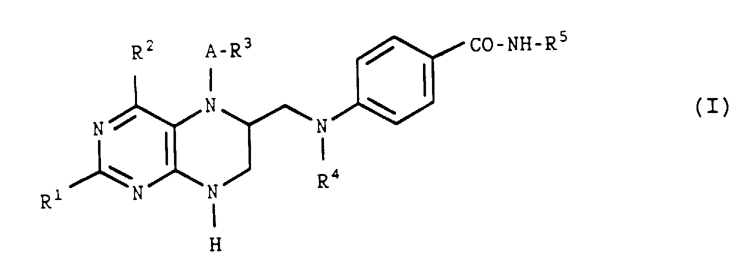 in der
- R¹: eine Amino-, C₁-C₆-Alkylamino-, Di-C₁-C₆-Alkylamino-oder eine durch eine Schutzgruppe substituierte Aminogruppe,
- R²: eine Hydroxy-, Amino-, C₁-C₆-Alkylamino-, Di-C₁-C₆-Alkylamino- oder eine durch eine Schutzgruppe substituierte Aminogruppe,
- R³: eine Formyl-, Carboxyl-, C₁-C₆-Alkoxycarbonyl-, Hydroxy-, Amino-, Mercapto- oder Aminocarbonylgruppe,
- R⁴: ein Wasserstoffatom oder ein C₁-C₆-Alkylgruppe bedeuten, und
- R⁵: einen α-Aminosäurerest darstellt, der über die α-Aminogruppe an die Carbonylgruppe gebunden ist und
- A: eine geradkettige oder verzweigte C₁-C₈-Alkylengruppe bedeutet, die durch ein Sauerstoff-, Schwefel- oder Stickstoffatom unterbrochen sein kann, und die gegebenenfalls durch Hydroxy- oder Oxogruppen substituiert ist,
sowie deren Tautomere, Ester und Amide, mit Ausnahme der Verbindungen 5-(Carboxymethyl)-5,6,7,8-tetrahydrofolsäure und 5-(5-Hydroxypentyl)-5,6,7,8-tetrahydrofolsäure.

Außerdem betrifft die Erfindung Immunogene, die aus den neuen Pteridin-Derivaten der Formel I hergestellt werden, wobei ein für immunologische Zwecke geeigneter Träger in 5-Stellung des Pteridin-Ringsystems an die erfindungsgemäßen Verbindungen gebunden ist.

Die Erfindung bezieht sich außerdem auf Antikörper, die mit Hilfe der oben genannten Immunogene erhältlich sind, sowie auf diagnostische Mittel zur Durchführung von immunologischen Bestimmungen, die diese Antikörper enthalten.

Gegenstand der Erfindung sind ferner Verfahren zur Herstellung der Pteridin-Derivate der Formel I sowie hierbei verwendete neue Zwischenprodukte.

Verbindungen mit dem Pteridin-Grundgerüst stellen eine wichtige Klasse von Verbindungen in der Biochemie dar. Wichtige Vertreter aus dieser Verbindungsklasse sind die Folsäure-Derivate, die eine physiologisch wichtige Rolle bei Stoffwechselprozessen im Organismus spielen. Sie sind Co-Faktoren oder Substrate bei einer Reihe von wichtigen Schlüsselenzymen in der DNS-Synthese bzw. in der Synthese von Purin- oder Pyrimidinbasen. Die physiologisch aktive Form der Folsäure ist die Tetrahydrofolsäure (FH₄) die als C₁-Gruppenüberträger bei der Synthese von Purinnukleotiden fungiert. Der Transport von Ein-Kohlenstoffkörper erfolgt hierbei über N¹⁰-Hydroxymethyl-, N^{5,10}-Methylen-tetrahydrofolsäure, N¹⁰-Formyl-tetrahydrofolsäure, N⁵-Formyltetrahydrofolsäure (Folinsäure) sowie anderen verschiedenen Zwischenstufen, die auf verschiedene Vorstufen bei der Synthese des Purinringsystems enzymatisch übertragen werden. Ist die Synthese von Purinnukleotiden gestört, so fehlen wesentliche Bausteine für die Bildung von DNA, die ihrerseits für die Zellteilung und Zellvermehrung von Bedeutung ist. Bei einer verminderten Purinsynthese entstehen oft pathologisch relevante, größere, kurzlebige Erythrozyten (Megalozyten). Dadurch kann die Bildung Thrombozyten oder Granulozyten gestört sein.

Bei der Störung der Purinbiosynthese kann es andererseits auch zur überproduktion von Purinbausteinen kommen, die ebenfalls negative Auswirkungen auf den Organismus haben. Dies kann ursächlich sein für eine Reihe von Krankheiten, wie z.B. Gicht. Gicht beruht auf einer erhöhten Harnsäurekonzentration (Hyperurikämie) im Blut. Die Harnsäure ist beim Menschen das Hauptabbauprodukt der Purinnukleotide. Häufig geht eine Überproduktion von Purinnukleotiden entsprechend mit einer erhöhten Konzentration des Abbauproduktes, der Harnsäure, einher, deren Konzentrationsanstieg im Blut auf eine Reihe von Krankheitsbildern schließen läßt, wie z.B. hämolytische Anämien, Neoplasien (Leukämie, chronische Myelosen, Lymphade-nosen), chronische Nephritis, verringerte Nierendurchblutung oder metabolische Azidosen.

Aus der Literatur sind einige Derivate der Folsäure bereits be-kannt. In J. Med. Chem. 1982, 25, 161 - 166 wird die Synthese von strukturanalogen Verbindungen beschrieben, die als potentielle Inhibitoren von folatabhängigen Enzymen in Frage kommen. Unter anderem werden darin auch die Verbindungen 5-(Carboxymethyl)-5,6,7,8-tetrahydrofolsäure und 5-(5-Hydroxypentyl)-5,6,7,8-tetra-hydrofolsäure beschrieben. Ferner ist die Herstellung von Folsäure und deren Salze aus DE-A-2,807,393 beschrieben.

Die Verbindung 5-Formyl-tetrahydrofolsäure (Folinsäure) findet Verwendung als pharmazeutischer Wirkstoff bei der Herstellung von Arzneimitteln zur Behandlung der megaloblastischen Folsäuremangel-Anämie, oder zur Behandlung von Autoimmunerkrankungen, wie beispielsweise Psoriasis oder der rheumatischen Arthritits, und ferner als Antidot zur Verstärkung der Verträglichkeit von Folsäure-Antagonisten in der Krebstherapie.

Eine weitere wichtige Verbindung aus der Klasse der Pteridin-Derivate sind Methotrexat-Derivate. Methotrexat wird als therapeutischer Wirkstoff in der antineoplastischen Therapie verwendet, wie z.B. bei der Behandlung von Leukämien. Das Wirkprinzip beruht darauf, daß die Purinbiosynthese gezielt gehemmt wird und damit eine vermehrte Zellteilung bzw. erhöhte Zellbildung unterbunden wird. Methotrexat ist ein häufig verwendeter Wirkstoff in der Krebstherapie, ebenso wie die entsprechenden Derivate des Methotrexats.

Aufgrund der oben beschrieben physiologischen Bedeutung von Pteridin-Derivaten und der therapeutischen Verwendung einiger Derivate ist es für die klinische Diagnostik von Bedeutung, die Konzentration derartiger Pteridin-Derivate in Körperflüssigkeiten zu bestimmen. In Anal. Biochem. 104, 1980, 347-354, wird ein Radioimmunoassay beschrieben, der zur Bestimmung von 5-Methyl-tetrahydrofolsäure dient. Das dort beschriebene Immunogen ist ein Konjugat aus 5-Methyltetrahydrofolsäure und Hämocyanin. In Immunochem. 1973, 10, 31-36, werden Immunokonjugate der Folinsäure beschrieben, wobei die Kopplung zwischen dem Hapten und γ-Globulin über die Carboxylgruppe des Glutamylrestes im Folin-säuremolekül erfolgt. Als Haptene werden Folinsäure, 5-Methyl-tetrahydrofolsäure (5-MTHF) und Folsäure benutzt. Außerdem sind entsprechende Konjugate der Folsäure mit Polypetiden aus Arch. Biochem. Biophys. 122, 1967, 157-163, bekannt, wobei die Kopplung ebenfalls über die Carboxylgruppe des Glutamylrestes erfolgt.

Die aus den obigen Literaturstellen bekannten Immunogene haben jedoch den Nachteil, daß bei deren Verwendung zur Herstellung von Antikörpern ausschließlich solche Antikörper erhalten werden, die eine relativ hohe Spezifität gegen das jeweilige als Immunogen eingesetzte Folsäure-Derivat besitzen. Pteridin-Derivate, insbesondere Folsäure-Derivate, liegen im Organismus sowohl in oxidierter Form als auch in der aktiven reduzierten Form als Di- oder Tetrahydrofolsäure-Derivate vor. Sie werden physiologisch schnell ineinander umgewandelt, so daß es für diagnostische Zwecke wichtig ist, die Summenkonzentration aus der oxidierten und reduzierten Form der Pteridin-Derivate zu bestimmen. Die Verwendung eines der aus dem Stand der Technik bekannten Antikörpers zur Entwicklung eines immunologischen Testes zur Bestimmung der Summenkonzentration der oxidierten und reduzierten Pteridin-Derivate ist mit Hilfe der bisher bekannten, sehr spezifisch wirkenden Antikörpern praktisch nicht möglich. Diese erkennen bevorzugt das jeweilige reduzierte oder oxidierte Pteridin-Derivat auf spezifische Weise, so daß bei der Bestimmung des Gesamtgehaltes von Pteridin-Derivaten mehrere Bestimmungsmethoden nebeneinander durchzuführen sind.

Theoretisch denkbar ist zwar die Verwendung eines Gemisches dieser bisher bekannten spezifischen Antikörpern, jedoch würde dies voraussetzen, daß der eine Antikörper das jeweilige Folsäure-Derivat, gegen das er gerichtet ist, ebensogut erkennt, wie der andere Antikörper das für ihn spezifische Folsäure-Derivat. Gleichzeitig müßten diese Antikörper zum jeweils anderen Antigen eine ähnlich hohe Kreuzreaktivität zeigen. Dies ist praktisch allerdings nicht realisierbar.

überraschenderweise wurde nun gefunden, daß sich mit Hilfe der neuen Pteridin-Derivate der Formel I, die in 5-Stellung des Ringsystems eine zur Kopplung an einen immunogenen Träger geeignete Gruppe besitzen, Antikörper herstellen lassen, die eine etwa gleichhohe Kreuzreaktivität sowohl gegen reduzierte als auch oxidierte Pteridin-Derivate zeigen. Durch die Kopplung dieser als Haptene geeigneten Pteridin-Derivate über das N⁵-Stickstoffatom an einen immunologischen Träger kommen offensichtlich die immunologischen Unterschiede zwischen der reduzierten und oxidierten Form der Pteridin-Derivate nicht in dem spezifischen Maße zum Ausdruck.

Erfindungsgemäß werden somit neue Pteridin-Derivate der Formel I zur Verfügung gestellt, wie sie in den Ansprüchen näher gekennzeichnet sind.

In der Formel I bedeutet R¹ insbesondere eine Aminogruppe und R² insbesondere eine Hydroxy- oder eine Aminogruppe. Besonders bevorzugt sind Verbindungen der allgemeinen Formel I, in der R¹ eine Aminogruppe bedeutet, R² eine Hydroxy- oder eine Aminogruppe bedeutet, und R³ eine Carboxylgruppe bedeutet.

Die Gruppe A-R³ steht für einen Substituenten in 5-Position am Pteridin-Grundgerüst, der als Brückenmolekül zur Bindung der Verbindungen der Formel I an einen immunologischen Träger geeignet ist. Dazu ist es erforderlich, daß dieser Brückensubstituent (Linker) eine chemisch reaktive Gruppe R³ besitzt, die sich zur Bildung von kovalenten Bindungen an den immunologischen Träger eignet. Derartige reaktive Gruppen sind beispielsweise Carboxylgruppen, die gegebenenfalls nach vorheriger Umsetzung mit einem Aktivierungsreagenz dazu geeignet sind, mit anderen chemisch reaktiven Gruppen, wie z.B. mit freien Aminogruppen von Proteinen, kovalente Bindungen zum immunologischen Träger auszubilden.

Je nach Art des als immunologischen Trägermoleküls eingesetzten Kopplungspartners kommen auch andere chemisch reaktive Gruppen in Frage. Soll bspw. die Kopplung über eine freie Carboxylgruppe des Trägermoleküls erfolgen, so werden als reaktive Gruppen am Pteridinsystem solche Substituenten eingesetzt, die vorzugsweise mit freien Carboxylgruppen reagieren, wie z.B. Amine. Enthalten die zu koppelnden immunologischen Träger freie Sulfhydrylgruppen, so kommen zur Kopplung über das Pteridin-Ringsystem als Gruppe R³ bspw. die Maleimidogruppe in Frage. Prinzipiell ist es auch möglich, die Kopplung zwischen den erfindungsgemäßen Pteridin-Derivaten und den immunologischen Trägern nicht unmittelbar, sondern mit Hilfe eines geeigneten homo- oder heterobifunktionellen Linkers, wie z.B. von DSS (Di-succinimidylsuberat), MMS (Maleinimido-hexansäure-N-hydroxy-succinimidester), SPDP (3'-(2'-Pyridyl-dithio)-propionsäure-N-hydroxysuccinimidester), MBS (3-Maleinimido-benzoesäure-N-hydroxysuccinimidester) oder Maleimido-ethylamin durchzuführen.

Als reaktive Gruppen R³ am Pteridin-System kommen insbesondere die Carboxylgruppe, eine Aminogruppe oder eine Sulfhydrylgruppe oder deren aktivierte Derivate in Frage. Die Gruppe R³ ist hierbei an einer beliebigen Stelle des als Brückenmolekül fungierenden Substituenten A gebunden. Bevorzugt erfolgt die Bindung von R³ allerdings endständig an die Gruppe A.

A kann eine geradkettige oder verzweigte C₁-C₈-Alkylenkette bedeuten die gegebenenfalls durch ein Sauerstoff-, Schwefel-oder Stickstoffatom unterbrochen sein kann, und die gegebenenfalls durch eine oder mehrere Hydroxy- oder Oxogruppen substituiert sein kann. In diesem Sinne kommen für A bspw. in Frage: Eine Methylen-, Ethylen-, n-Propylen-, n-Butylen-, Isobutylen-, Pentylen-, Hexylen-, 1-Hydroxyethylen-, 1-Hydroxy-n-propylen-, Ethoxy-methylen-, Ethoxyethylen-, Methylencarbonyl- oder Ethylencarbonylgruppe. Bevorzugt ist A eine C₂-C₈-Alkylengruppe.

Für den Fall, daß R³ eine Carboxylgruppe bedeutet, wird diese vorzugsweise zunächst mit einem Aktivierungsreagenz, wie z.B. N-Hydroxysuccinimid, Carbonyldiimidazol, N-Ethyl-N'-3 (Dimethyl-aminopropyl)-carbodiimid umgesetzt, bevor die Kopplung an den immunologischen Träger erfolgt.

R⁵ steht für einen neutralen, sauren oder basischen Aminosäurerest, wobei die Bindung an die Carbonylgruppe der Pteridinderivate über die alpha-Aminogruppe des Aminosäurerestes erfolgt. Als Aminosäurereste kommen beispielsweise der Glutamyl-, Lysin-, Arginin- oder Ornithinrest in Frage. Jedoch können auch andere beliebige Aminosäurereste analog gebunden werden. Die Aminosäurereste besitzen ein chirales C-Atom und können in der optisch aktiven R- oder S-Form, oder auch als Racemat vorliegen. Bevorzugt ist jedoch die L-Form des Aminosäurerestes.

Bevorzugte Pteridin-Derivate der Formel I sind solche in denen R¹ eine Aminogruppe, R² eine Amino- oder Hydroxygruppe und R⁴ ein Wasserstoffatom oder die Methylgruppe bedeutet.

Für R⁵ kommt vorzugsweise der Glutamylrest in Frage. Enthalten die Aminosäure-reste zusätzliche Carboxylgruppen, so können diese sowie die α-ständige Carboxylgruppen der Aminosäure in freier Form oder in Form der Amide, Ester oder Salze vorliegen.

Bevorzugter Rest für R⁵ ist insbesondere die Gruppe -CH(COOH)-CH₂-CH₂-COOH und -CH(CONH₂)-CH₂-CH₂-CONH₂.

Die "Alkyl"-Teile bei den in R¹ - R⁵ genannten Gruppen können in allen Fällen geradkettig oder verzweigt sein und 1 - 6 C-Atome enthalten, wie z.B. die Methyl, Ethyl oder Isopropylgruppe.

Die Herstellung der erfindungsgemäßen Verbindungen der Formel I erfolgt dadurch, daß man eine Verbindung der Formel II 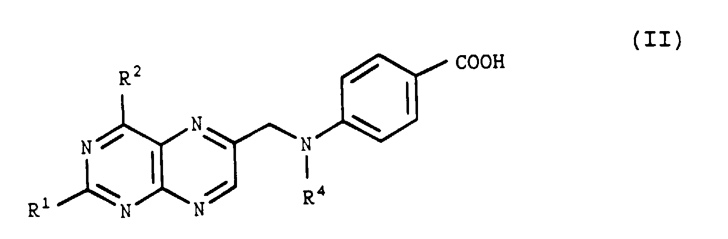 in der vorhandene Aminogruppen gegebenenfalls durch geeignete Schutzgruppen geschützt sind, mit einem die Carboxylgruppe aktivierenden Reagenz umsetzt, und anschließend die so erhaltenen Verbindungen mit einer Verbindung der Formel III

R⁵-NH₂ (III)

in der R⁵ die oben angegebene Bedeutung besitzt, zu Verbindungen der Formel IV 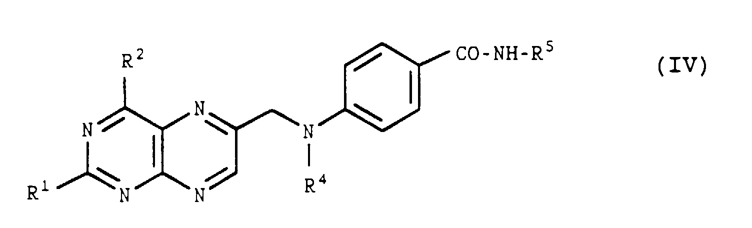 umsetzt, und anschließend die so erhaltene Verbindung der Formel IV mit einem geeigneten Reduktionsmittel reduziert, und diese dann mit einer Verbindung der Formel V

OHC-A-R³ (V)

umsetzt, anschließend reduziert und gegebenenfalls vorhandene Schutzgruppen wieder abspaltet und Verbindungen der Formel I isoliert.

Verbindungen der Formel II, in denen R¹ oder R² eine Aminogruppe darstellt, werden vor der Aktivierung der Carboxylgruppe zweckmäßiger Weise mit geeigneten Aminoschutzgruppenreagenzien umgesetzt, wie z.B. Trifluoressigsäureanhydrid. Die Aktivierung der Carboxylgruppe erfolgt mit an sich bekannten Aktivierungsreagenzien, wie z.B. Chlorameisensäureestern und ähnlichen Derivaten. Als Lösungsmittel eignen sich vorzugsweise DMF sowie andere organische Lösungsmittel.

Verbindungen der Formel II mit aktivierter Carboxylgruppe werden dann mit α-Aminosäure-Derivaten der Formel III umgesetzt. Für den Fall, daß diese in der Seitengruppe funktionelle Gruppen enthalten, wie z.B. eine Carboxylgruppe im Fall der Glutamin- oder Asparaginsäure, werden diese Verbindungen der Formel III vorzugsweise in Form ihrer geschützen Derivate, wie z.B. der Amide eingesetzt.

Die Reduktion von Verbindungen der Formel IV und Umsetzung mit den Aldehyden der Formel V erfolgt nach an sich bekannten Methoden (Blair, Analytical Biochemistry, 41, 332 - 337, 1971).

Verbindungen der Formel I enthalten zwei chirale Kohlenstoffatome, von denen ein asymmetrisches Zentrum im Aminosäurerest R⁵ enthalten ist, während das durch Hydrierung des Pteroylrestes entstandene zweite chirale C-Atom in Position 6 des Pteridinrings vorliegt. Werden bei der Synthese racemische Aminosäuren R⁵-NH₂ eingesetzt, so bestehen die Verbindungen der Formel I in der Regel aus einem Gemisch von zwei Diastereomerenpaaren. Wird hingegen entweder die L- oder D- Aminosäure eingesetzt, so entstehen entsprechend 1:1-Gemische von zwei Diastereomeren. Die Auftrennung dieser Diastereomeren kann nach an sich bekannten Methoden durchgeführt werden, z.B. durch fraktionierte Kristallisation oder durch chromatographische Methoden.

Gegenstand der vorliegenden Erfindung sind insbesondere auch neue Konjugate der Formel VI 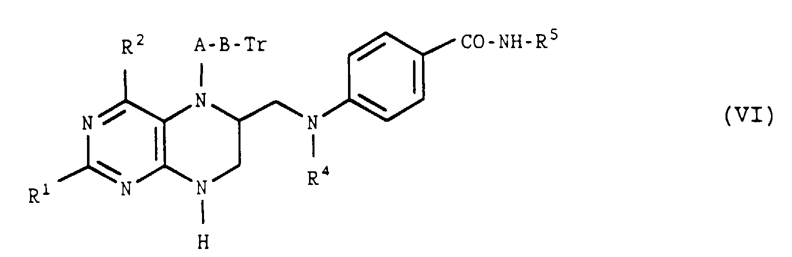 in der R¹, R², R⁴, R⁵ und A die oben angegebenen Bedeutungen besitzen und B eine als kovalente Brücke fungierende Gruppe zwischen dem Pteridin-Derivat und dem immunologischen Träger Tr ist. Diese Konjugate können als Immunogene bei der Erzeugung von Antikörpern eingesetzt werden.

Für den Fall, daß R³ in der Formel I eine Carboxylgruppe darstellt, ist B bspw. die Gruppe -CO-NH-, die eine freie Aminogruppe des immunologischen Trägers Tr mit dem Pteridin-Ringsystem verbindet (-A-CO-NH-Tr). Ist R³ eine Aminogruppe, so stellt B bspw. die Gruppe -NH-CO- dar, die als Brücke zu einer freien Carboxylgruppe des immunologischen Trägers Tr fungiert (-A-NH-CO-Tr). Falls R³ eine Mercaptogruppe bedeutet, so ist B ein Schwefelatom, das das Pteridinringsystem mit dem immunologischen Träger verbindet, der gegebenenfalls zuvor mit sulfhydrylgruppen-spezifischen Reagenzien, wie z.B. der Maleimidogruppe, modifiziert worden ist. B kann prinzipiell auch einen homo- oder heterobifunktionellen Linker darstellen, der das Pteridin-Derivat mit dem Träger Tr verbindet.

Als immunologische Träger kommen bspw. Peptide oder Proteine in Frage, wie z.B. Galactosidase, Rinderserumalbumin (BSA), Keyhole limpet hemocyanine (KLH), Edestin oder Immunglobuline. Tr kann aber auch einen festen polymeren Träger darstellen, wie beispielsweise Aminosepharose.

Bevorzugte Immunogene der Formel VI sind insbesondere solche, in denen A eine C₁-C₈-Alkylenkette, vorzugsweise C₃-C₅-Alkylen bedeutet. B bedeutet vorzugsweise die Gruppen -CONH-oder -NHCO-. Für R⁵ ist der Glutamylrest -CH(COOH)-CH₂-CH₂-COOH oder -CH(CONH₂)-CH₂-CH₂-CONH₂ bevorzugt. R¹ kann eine Hydroxy- oder bevorzugt eine Aminogruppe bedeuten. Besonders bevorzugte Immunogene der Formel VI sind solche, in denen R¹ eine Aminogruppe, R² eine Hydroxy- oder Aminogruppe, R⁴ ein Wasserstoff oder eine C₁-C₆-Alkylgruppe und R⁵ den Aminosäurerest -CH(COOH)-CH₂-CH₂-COOH oder -CH(CONH₂)-CH₂-CH₂-CONH₂, A eine C₁-C₈-Alkylenkette und B eine -CO-NH- oder -NH-CO-Gruppe bedeutet.

Ferner betrifft die vorliegende Erfindung auch neue Antikörper, die mit Hilfe der erfindungsgemäßen Immunogene der Formel VI hergestellt werden. Diese werden nach an sich bekannten Immunisierungsverfahren hergestellt.

Die Konjugate der Formel VI können direkt für die Immunisierung eingesetzt werden. Hierbei wird das Konjugat in den zur Antikörperbildung geeigneten Organismus in Abständen mehrmals injiziert. Es kommt dabei zur Bildung von Antikörpern, die gegen das verwendete Konjugat gerichtet sind. Die Antikörper werden dann in an sich bekannter Weise aus dem Organismus isoliert und gereinigt. Die erfindungsgemäßen Konjugate der Formel VI eignen sich sowohl zur Immunisierung für die Herstellung polyklonaler als auch zur Herstellung monoklonaler Antikörper.

Überraschenderweise besitzen die erfindungsgemäßen Antikörper eine etwa gleich hohe Kreuzreaktivität gegen die Verbindungen der Formel I in oxidierter bzw. reduzierter Form. Damit sind sie insbesondere dazu geeignet, die Summenkonzentration von bspw. Folsäure-Derivaten und deren 5,8-Dihydro- bzw. 5,6,7,8-Tetrahydro-Derivaten zu bestimmen.

Demgemäßbezieht sich die vorliegende Erfindung ebenfalls auf diagnostische Mittel, die die neuen erfindungsgemäßen Antikörper enthalten, sowie auf deren Verwendung zur Herstellung von immunologischen Diagnosekits.

Die folgenden Beispiel erläutern die Erfindung anhand einiger repräsentativer Ausführungsbeispiele:

### Beispiel 1

### Bis(trifluoracetyl)pteroinsäure

4.68 g (15 mmol) Pteroinsäure werden solange in 150 ml Trifluoressigsäureanhydrid unter Rückfluß gerührt, bis vollständige Lösung eintritt (ca. 20 h). Danach wird das überschüssige Anhydrid und die entstandene Triflouressigsäure abgedampft und der Rückstand mit Eiswasser digeriert. Der Feststoff wird abgesaugt und im Exsikkator über Phosphorpentoxid getrocknet.
Ausbeute: 6.7 g (89 % d.Th.).
DC: Kieselgel, Essigester/Eisessig 9:1, R_{f}= 0.65 .

### Beispiel 2

### Bis(trifluoracetyl)pteroinsäure-kohlensäureisobutylesteranhydrid

5.0 g (10 mmol) Bis(trifuoracetyl)pteroinsäure werden in 150 ml absolutem DMF gelöst und mit 2.05 ml Triethylamin versetzt. Zu der Lösung gibt man 1.82 ml Chlorameisensäureisobutylester und läßt 3 h bei Raum-temperatur rühren. Die Lösung des gemischten Anhydrids der Titelverbindung wird ohne weitere Aufreinigung zur nächsten Stufe eingesetzt.

### Beispiel 3

### Bis(trifluoracetyl)folsäurediamid

Die Lösung des Anhydrids der in Beispiel 2 hergestellten Verbindung wird mit 2.25 g L-Glutaminsäurediamid-Hydrochlorid versetzt und 20 h bei Raumtemperatur gerührt. Das ausgefallene Ammoniumsalz wird abgesaugt und das Filtrat im Vakuum bei 40°C eingedampft. Der ölige Rückstand wird mit 1 %iger Essigsäure digeriert und abgesaugt. Man wäscht mit Wasser nach und trocknet das Produkt im Exsikkator über Phosphorpentoxid.
Ausbeute: 5.6 g (89 % d.Th. bezogen auf die Verbindung aus Beispiel 1).
DC: Kieselgel, Essigester/Eisessig 9:1; R_{f}= 0.50 .

### Beispiel 4

### N⁵-(Carboxypropyl)folsäurediamid

1.26 g des Bis(trifluoracetyl)folsäurediamids werden in 20 ml aminfreiem DMF gelöst, mit 40 ml Wasser verdünnt und portionsweise mit 1.5 g Natriumborhydrid versetzt. Nach 2 h Rühren bei Raumtemperatur gibt man langsam gerade soviel 50 %iger Essigsäure zu, bis keine Gasentwicklung mehr zu beobachten ist (ca. pH 8.0) Dann versetzt man mit 9.6 ml 15 %iger wässriger Bernsteinsäurealdehydlösung, stellt mit 10 n NaOH auf pH = 7.8 und läßt 10 min rühren. Anschließend werden wiederum 1.5 g Natriumborhydrid in 10 ml Wasser langsam zugetropft, wobei leichte Erwärmung auf ca. 40°C zu beobachten ist. Nach 1 h stellt man den pH-Wert mit 50 %iger Essigsäure wieder auf 7.8 und gibt erneut 9.6 ml Bernsteinsäurealdehydlösung und 1.5 g Natriumborhydrid analog oben beschriebener Prozedur zu. Nach beendeter Zugabe und 1 h Rühren bei Raumtemperatur wird mit 50 %iger Essigsäure auf pH 6.5 gestellt, über einen engporigen Papierfilter filtriert und das Filtrat im Vakuum eingedampft. Der Eindampfrückstand wird in 80 ml N₂-gesättigtem Wasser gelöst und auf eine Säule (2 x 30 cm) mit 80 g Aktivkohle gegeben. Man wäscht mit 100 ml N₂-gesättigtem Wasser und eluiert anschließend das Produkt mit 600 ml einer N₂-gesättigten Mischung aus Methanol/Wasser/Ammoniak-Lösung (80/18/2 v/v/v). Die Lösung wird im Vakuum bei 45°C auf ca. 80 ml eingeengt, mit Eisessig bis pH = 5.0 angesäuert und dann lyophilisiert.
Ausbeute: 0.6 g (51 % d.Th.).
DC: Kieselgel, Butanol/Eisessig/Wasser (40/10/50 v/v/v) + 0.1 % Mercaptoethanol; R_{f}= 0.25 .

### Beispiel 5

### N⁵-(Carboxypropyl)folsäurediamid-β-Galaktosidase-Konjugat

95 mg N⁵-(Carboxypropyl)folsäurediamids werden zu einer Lösung von 170 mg β-Galaktosidase in 5 ml Wasser gegeben und mit 20 mg N-Hydroxysuccinimid und 37 mg N-Ethyl-N'-(dimethylamino-propyl)-carbodiimid-Hydrochlorid versetzt. Man läßt 16 h bei Raumtemperatur rühren und gibt die Lösung dann auf eine Säule (2.5 x 30 cm) mit Ultrogel^{R} ACA 202, äquilibriert mit 0.01 m Kaliumphosphat-Puffer pH 7.5 + 0.9 % NaCl. Nach Elution mit dem gleichen Puffergemisch erhält man eine Lösung von 200 mg Hapten-Protein-Konjugat (Titelverbindung). Die Bestimmung der Ausbeute erfolgt hierbei durch Messung der UV-Absorption bei ) = 280 nm im Vergleich mit einer Proteinstandardlösung.

## Patentansprüche

1. Pteridin-Derivate der Formel I 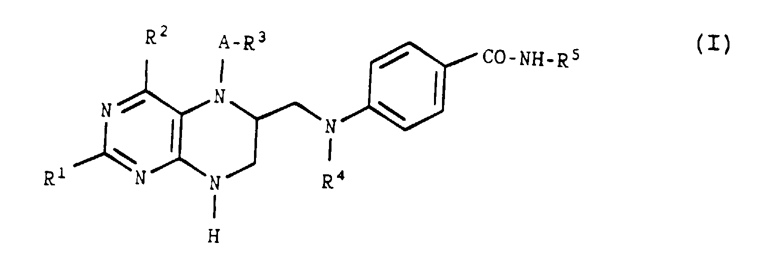 in der
R¹ eine Amino, C₁-C₆-Alkylamino, Di-C₁-C₆-Alkylamino oder eine durch eine Schutzgruppe substituierte Aminogruppe,
R² eine Hydroxy-, Amino-, C₁-C₆-Alkylamino-, Di-C₁-C₆-Alkylamino- oder eine durch eine Schutzgruppe substituierte Aminogruppe,
R³ eine Formyl-, Carboxy-, C₁-C₆-Alkoxycarbonyl-, Hydroxy-, Amino-, Mercapto- oder Aminocarbonylgruppe,
R⁴ ein Wasserstoffatom oder ein C₁-C₈-Alkylgruppe bedeuten, und
R⁵ einen α-Aminosäurerest darstellt, die über die α-Aminogruppe an die Carbonylgruppe gebunden ist, und
A eine geradkettige oder verzweigte C₁-C₈-Alkylengruppe bedeutet, die durch ein Sauerstoff-, Schwefel- oder Stickstoffatom unterbrochen sein kann, und die gegebenenfalls durch Hydroxy- oder Oxogruppen substituiert ist,
sowie deren Ester und Amide, mit Ausnahme der Verbindungen 5-(Carboxymethyl)-5,6,7,8-tetrahydrofolsäure und 5-(5-Hydroxypentyl)-5,6,7,8-tetrahydrofolsäure.

2. Pteridin-Derivate der Formel I gemäß Anspruch 1, dadurch gekennzeichnet, daß R¹ eine Aminogruppe bedeutet, R² eine Hydroxy- oder Aminogruppe bedeutet, und R³ eine Carboxylgruppe darstellt.

3. Pteridin-Derivate der Formel I gemäß Anspruch 1, dadurch gekennzeichnet, daß R⁴ ein Wasserstoffatom darstellt.

4. Pteridin-Derivate der Formel I gemäß Anspruch 1, dadurch gekennzeichnet, daß R⁵ die Gruppe -CH(COOH)-CH₂-CH₂-COOH oder -CH(CONH₂)-CH₂-CH₂-CONH₂ bedeutet.

5. Pteridin-Derivate der Formel I gemäß Anspruch 1, dadurch gekennzeichent, daß A eine C₂-C₈-Alkylengruppe bedeutet.

6. Verfahren zur Herstellung von Pteridin-Derivaten gemäß einem der Ansprüche 1-5, dadurch gekennzeichent, daß man eine Verbindung der Formel IV 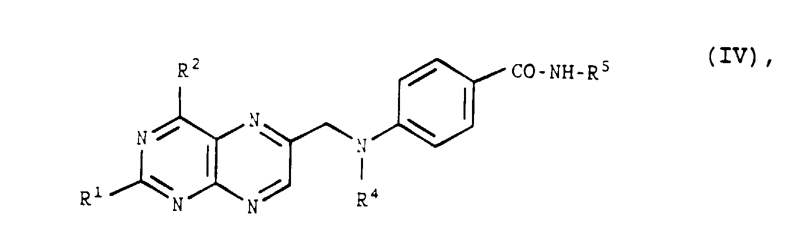 mit einem geeigneten Reduktionsmittel zunächst in die entsprechenden Di- oder Tetrahydroverbindungen überführt und diese anschließend mit einer Verbindung der Formel V
CHO-A-COR³ (V),
umsetzt, anschließend mit einem geeigneten Reduktionsmittel reduziert und gegebenenfalls vorhandene Schutzgruppen wieder abspaltet und Verbindungen der Formel I isoliert.

7. Verwendung von Pteridin-Derivaten der Formel I gemäß einem der Ansprüche 1-5 zur Herstellung von Immunogenen.

8. Immunogene der Formel VI 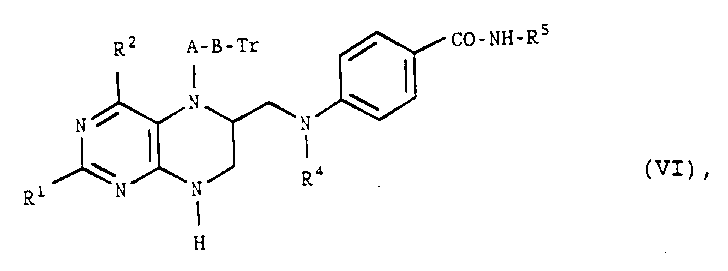 in der R¹, R², R⁴, R⁵ und A die in Anspruch 1 angegebene Bedeutungen besitzen und
B eine als kovalente Brücke fungierende Gruppe, und
Tr ein immunologischer Träger ist.

9. Immunogene gemäß Anspruch 8, dadurch gekennzeichnet, daß R¹ eine Aminogruppe, R² eine Hydroxy- oder Aminogruppe, R⁴ ein Wasserstoff oder eine C₁-C₆-Alkylgruppe und R⁵ den Aminosäurerest -CH(COOH)-CH₂-CH₂-COOH oder -CH(CONH₂)-CH₂-CH₂-CONH₂, A eine C₁-C₈-Alkylenkette und B eine -CO-NH- oder -NH-CO-Gruppe bedeutet.

10. Immunogene gemäß Anspruch 8 oder 9, dadurch gekennzeichnet, daß der immunologische Träger Tr das Enzym ß-Galactosidase ist.

11. Diagnostisches Mittel enthaltend Immunogene gemäß Anspruch 8, 9 oder 10 zur Durchführung von immunologischen Bestimmungen.

12. Verwendung von Immunogenen gemäß einem der Ansprüche 8 bis 10 zur Herstellung von Antikörpern.

13. Antikörper erhältlich durch Immunisierung von Säugern unter Verwendung von Immunogenen gemäß einem der Ansprüche 8-10.

14. Arzneimittel enthaltend mindestens eine Verbindung der Formel I gemäß einem der Ansprüche 1-5 oder deren pharmakologisch verträgliche Salze sowie übliche pharmazeutische Träger- und/oder Hilfsstoffe, insbesondere zur Behandlung von Krebs.

## Claims

1. Pteridine derivatives having the formula I 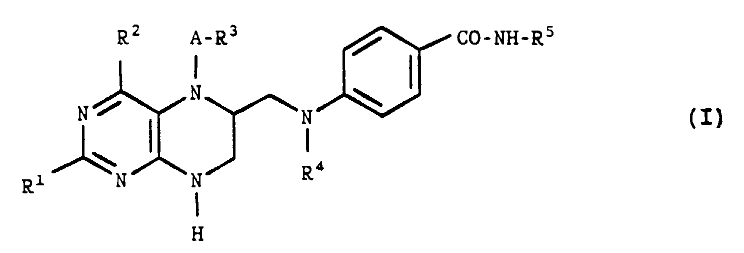 in which
R¹ denotes an amino, C₁-C₆ alkylamino, di-C₁-C₆ alkylamino or an amino group substituted by a protective group,
R² denotes a hydroxy group, amino group, C₁-C₆ alkylamino group, di-C₁-C₆ alkylamino group or an amino group substituted by a protective group,
R³ denotes a formyl group, carboxyl group, C₁-C₆ alkoxycarbonyl group, hydroxy group, amino group, mercapto group or aminocarbonyl group,
R⁴ denotes a hydrogen atom or a C₁-C₈ alkyl group and
R⁵ represents an α-amino acid residue which is bound to the carbonyl group via the α-amino group and
A denotes a straight-chained or branched C₁-C₈ alkylene group in which an oxygen atom, sulphur atom or nitrogen atom may be inserted and which is substituted, if desired, by hydroxy groups or oxo groups,
as well as their esters and amides with the exception of the compounds 5-(carboxymethyl)-5,6,7,8-tetrahydrofolic acid and 5-(5-hydroxypentyl)-5,6,7,8-tetrahydrofolic acid.

2. Pteridine derivatives having the formula I as claimed in claim 1, wherein R¹ denotes an amino group, R² represents a hydroxy group or an amino group and R³ represents a carboxyl group.

3. Pteridine derivatives having the formula I as claimed in claim 1, wherein R⁴ represents a hydrogen atom.

4. Pteridine derivatives having the formula I as claimed in claim 1, wherein R⁵ denotes the group -CH(COOH) -CH₂-CH₂-COOH or -CH(CONH₂)-CH₂-CH₂-CONH₂.

5. Pteridine derivatives having the formula I as claimed in claim 1, wherein A denotes a C₂-C₈ alkylene group.

6. Process for the production of pteridine derivatives as claimed in one of the claims 1 - 5, wherein a compound having the formula IV 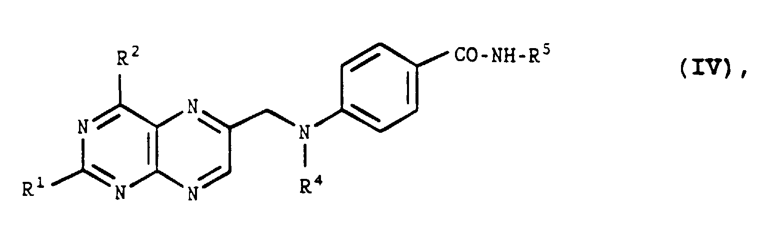 is first converted into the corresponding dihydro or tetrahydro compounds with a suitable reducing agent and these are subsequently reacted with a compound having the formula V
CHO-A-COR³ (V),
subsequently reduced with a suitable reducing agent and protective groups which may be present are again cleaved off and compounds having the formula I are isolated.

7. Use of pteridine derivatives having the formula I as claimed in one of the claims 1 - 5 for the production of immunogens.

8. Immunogens having the formula VI 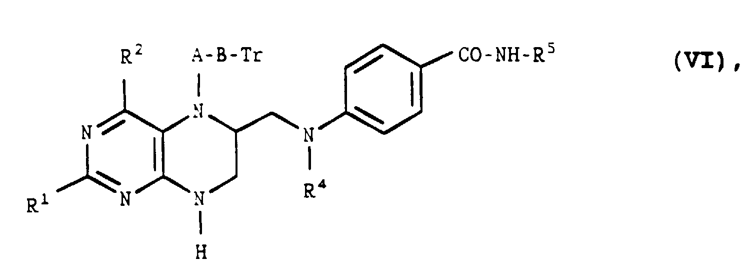 in which R¹, R², R⁴, R⁵ and A have the meanings stated in claim 1 and
B is a group acting as a covalent bridge and
Tr is an immunological carrier.

9. Immunogens as claimed in claim 8, wherein R¹ denotes an amino group, R² denotes a hydroxy group or an amino group, R⁴ denotes hydrogen or a C₁-C₆ alkyl group and R⁵ denotes the amino acid residue -CH(COOH)-CH₂-CH₂ -COOH or -CH(CONH₂)-CH₂-CH₂-CONH₂, A denotes a C₁-C₈ alkylene chain and B denotes a -CO-NH- or -NH-CO-group.

10. Immunogens as claimed in claim 8 or 9, wherein the immunological carrier Tr is the enzyme β-galactosidase.

11. Diagnostic agent containing immunogens as claimed in claim 8, 9 or 10 for carrying out immunological determinations.

12. Use of immmunogens as claimed in one of the claims 8 to 10 for the production of antibodies.

13. Antibodies obtainable by immunizing mammals using the immunogens as claimed in one of the claims 8 - 10.

14. Pharmaceutical agent containing at least one compound of formula I as claimed in one of the claims 1-5 or pharmacological compatible salts thereof as well as usual pharmaceutical carrier and/or auxiliary agents, in particular for a treatment of cancer.

## Revendications

1. Dérivés de pteridine de formule I 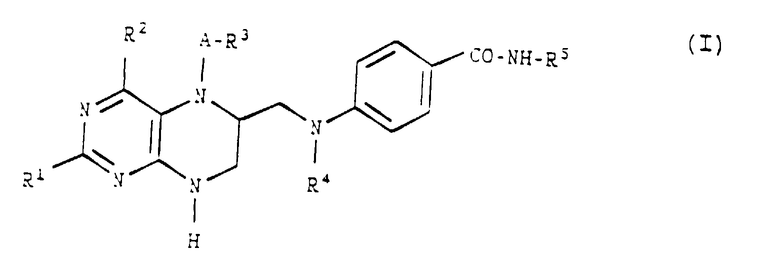 dans laquelle
R¹ représente un groupe amino, (alkyle en C₁-C₆)amino, di(alkyle en C₁-C₆)amino, ou un groupe amino substitué par un groupe protecteur,
R² représente un groupe hydroxy, amino, (alkyle en C₁-C₆)amino, di(alkyle en C₁-C₆)amino, ou un groupe amino substitué par un groupe protecteur,
R³ représente un groupe formyle, carboxyle, (alcoxy en C₁-C₆)-carbonyle, hydroxy, amino, mercapto ou aminocarbonyle,
R⁴ représente un atome d'hydrogène ou un groupe alkyle en C₁-C₈, et
R⁵ représente un résidu d'acide α-aminé, qui est lié au groupe carbonyle par l'intermédiaire du groupe α-amino, et
A représente un groupe alkylène linéaire ou ramifié en C₁-C₈, qui peut être interrompu par un atome d'oxygène, de soufre ou d'azote, et qui est éventuellement substitué par des groupes hydroxy ou oxo,
ainsi que leurs esters et amides, à l'exception des composés que sont l'acide 5-(carboxyméthyl)-5,6,7,8-tétrahydrofolique et l'acide 5-(5-hydroxypentyl)-5,6,7,8-tétrahydrofolique.

2. Dérivés de pteridine de formule I selon la revendication 1, caractérisés par le fait que R¹ représente un groupe amino, R² représente un groupe hydroxy ou amino et R³ représente un groupe carboxyle.

3. Dérivés de pteridine de formule I selon la revendication 1, caractérisés par le fait que R⁴ représente un atome d'hydrogène.

4. Dérivés de pteridine de formule I selon la revendication 1, caractérisés par le fait que R⁵ représente un groupe -CH(COOH)-CH₂-CH₂-COOH ou -CH(CONH₂)-CH₂-CH₂-CONH₂.

5. Dérivés de pteridine de formule I selon la revendication 1, caractérisés par le fait que A représente un groupe alkylène en C₂-C₈.

6. Procédé de préparation de dérivés de pteridine selon l'une quelconque des revendications 1-5, caractérisé par le fait que l'on transforme d'abord un composé de formule IV 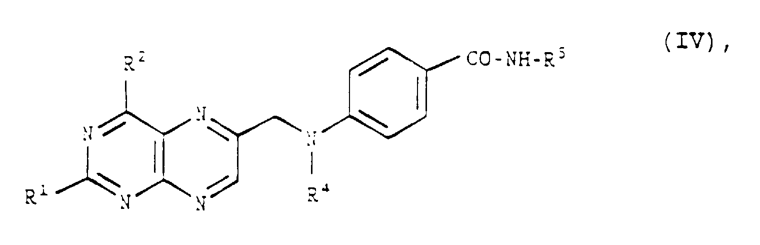 à l'aide d'un agent réducteur approprié, en le composé di- ou tétrahydro correspondant et ensuite, on fait réagir celui-ci avec un composé de formule V
CHO-A-COR³ (V),
puis on le réduit à l'aide d'un agent réducteur approprié, on sépare à nouveau les groupes protecteurs éventuellement présents et on isole le composé de formule I.

7. Utilisation de dérivés de pteridine de formule I selon l'une quelconque des revendications 1-5, pour la préparation d'immunogènes.

8. Immunogènes de formule VI 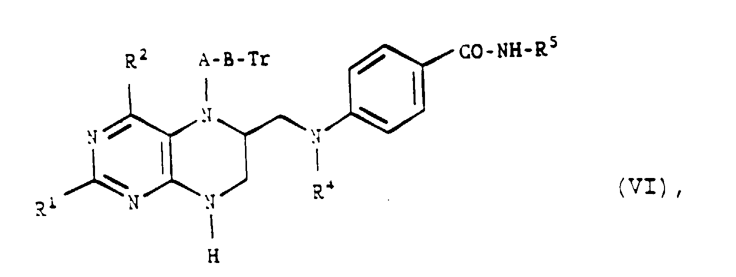 dans laquelle R¹, R², R⁴, R⁵ et A ont les significations indiquées à la revendication 1 et
B représente un groupe constituant un pont covalent et
Tr représente un véhicule immunologique.

9. Immunogènes selon la revendication 8, caractérisés par le fait que R¹ représente un groupe amino, R² représente un groupe hydroxy ou amino, R⁴ représente un atome d'hydrogène ou un groupe alkyle en C₁-C₆ et R⁵ représente le résidu d'acide aminé -CH(COOH)-CH₂-CH₂- COOH ou -CH(CONH₂)-CH₂-CH₂ CONH₂, A représente une chaîne alkylène en C₁-C₈ et B, un groupe -CO-NH- ou -NH-CO-.

10. Immunogènes selon la revendication 8 ou 9, caractérisés par le fait que le véhicule immunologique Tr est l'enzyme β-galactosidase.

11. Agent de diagnostic contenant des immunogènes selon la revendication 8, 9 ou 10, destiné à la réalisation de déterminations immunologiques.

12. Utilisation d'immunogènes selon l'une quelconque des revendications 8 à 10, pour la préparation d'anticorps.

13. Anticorps pouvant être obtenus par immunisation de mammifères en utilisant des immunogènes selon l'une quelconque des revendications 8 à 10.

14. Médicament contenant au moins un composé de formule I selon l'une quelconque des revendications 1 à 5 ou un de ses sels pharmacologiquement acceptables, ainsi que des véhicules et/ou adjuvants pharmaceutiques usuels, destiné en particulier au traitement de cancers.
